# EUROPÄISCHE PATENTSCHRIFT

(11) **EP 1 267 163 B1**
(45) Veröffentlichungstag und Bekanntmachung des Hinweises auf die Patenterteilung: **07.05.2008**
(21) Anmeldenummer: 02012966.4
(22) Anmeldetag: 12.06.2002
(51) Int. Cl.: G01N 33/34

(54) **Prüfgerät zur fotoelektrischen Prüfung des Leimungsgrades und der Saugfähigkeit**
Test device for photoelectrically measuring the sizing degree and the absorbency
Appareil d'essai pour mesurer photoélectriquement le degré de collage et la capacité d'absorption

(30) Priorität: 15.06.2001 DE 10128643
(43) Veröffentlichungstag der Anmeldung: 18.12.2002
(73) Patentinhaber: IGT emus GmbH, 04177 Leipzig (DE)
(72) Erfinder: Portner, Wolfgang, Dipl.-Ing., 04275 Leipzig (DE); Gross, Thomas, Dipl.-Ing., 04416 Markkleeberg (DE)
(74) Vertreter: Borchard, Wolfgang

(56) Entgegenhaltungen:
- EP-A- 0 677 730
- DD-A- 20 498
- US-A- 5 822 070

## Beschreibung

Die Erfindung betrifft ein Prüfgerät zur fotoelektrischen Prüfung des Leimungsgrades und der Saugfähigkeit von Proben aus Papier, Pappe, Filtermaterial oder einem anderen saugfähigen Material mit Hilfe einer Prüfflüssigkeit und unter Verwendung einer horizontal in einer Prüfebene angeordneten Probe, die mit einem elastischen Druckring festgehalten wird.

Bei einem bekannten fotoelektrischen und in dem DD WP 20 498 beschriebenen Gerät mit Zwischenwertablesung zur fotoelektrischen Prüfung des Leimungsgrades und der Saugfähigkeit von Papier mit Hilfe einer Prüfflüssigkeit wird ein mit Prüfflüssigkeit gefüllter Behälter und ein stets gleichmäßig gefülltes Schöpfgefäß eingesetzt. An das Schöpfgefäß wird eine Materialprobe heran geführt, die in ihrer Lage durch einen am Umfang wirkenden, elastischen Druckring gehalten wird. Die Materialprobe wird mit der geschöpften Prüfflüssigkeit benetzt, die sich im Unterteil des Gerätes befindet. In der Mitte einer Auflageplatte für die Materialprobe befindet sich eine Öffnung, über der eine ringförmige Fotozelle angeordnet ist. Das von einer Lichtquelle ausgehende Licht fällt durch die Öffnung auf die Oberseite der Materialprobe, von der es auf die untere Seite der Fotozelle reflektiert wird. Eine zunehmende Schwärzung der Materialprobe durch die absorbierte Flüssigkeit wird gemessen und in Relation mit der Saugfähigkeit gebracht.

Dokument EP0677730 offenbart ein Prüfgerät zur fotoelektrischen Prüfung des Leimungsgrades und der Saugfähigkeit von Proben aus Papier, Pappe, Filtermaterial oder einem anderen saugfähigen Material mit Hilfe einer Prüfflüssigkeit sowie unter Verwendung einer horizontal in einer Prüfebene angeordneten und mit einem elastischen Druckring gehaltenen Probe, gekennzeichnet durch einen ortsfesten, über eine Zulaufleitung mit einer Mikrodosierpumpe verbundenen Meniskuszylinder für die Prüfflüssigkeit, wobei bei Inbetriebnahme der Mikrodosierpumpe der Meniskuszylinder mit einem aus überlaufender Prüfflüssigkeit bestehenden Meniskus versehen und die Probe auf dem Meniskus schwimmend angeordnet ist.

Über der Auflageplatte befindet sich die elektrische Lichtquelle mit einer Lampe, die im Verhältnis zur der geringen Menge an Prüfflüssigkeit eine nicht zu vernachlässigende Leistungsaufnahme und Wärmeentwicklung aufweist. Die erzeugte Wärme führt zu einer Temperaturerhöhung der Prüfflüssigkeit in dem Schöpfgefäß, die auch nach beendigter Messung und nach dem Herunterfahren sowie dem Schöpfen von neuer Prüfflüssigkeit nicht vollständig ausgeglichen wird. Bei Dauermessungen führt diese Aufheizung der Prüfflüssigkeit zu kürzeren Durchschlagzeiten der Prüfflüssigkeit, die das Meßergebnis verfälschen. In der Praxis wurde daher entsprechend der Veröffentlichung in der Zeitschrift "Das Papier", Heft 6 1984, Seite 262 - 266 versucht, diesem Nachteil mit einer als Kaltlichtlampe bezeichneten Lichtquelle abzuhelfen, die mit einer Halogenlampe ausgerüstet ist. Der Transport des Lichts erfolgt mit einem Glasfaserstrang als Lichtleiter. Nachteilig ist der hohe konstruktive Aufwand und die Bündelung des Lichts, das über der Materialprobe gleichmäßig verteilt werden muß.

Erfindungsgemäß ist eine elektrische Lichtquelle vorgesehen, die durch einen in Folienform ausgebildeten Elektrolumineszenz - Leuchtkörper gebildet wird. Elektrolumineszenz - Leuchtkörper sind an sich bekannt. Sie sind in Folienform aufgebaut, die eine Frontelektrode aus transparentem Material, eine Leuchtstoffschicht, eine Isolationsschicht und eine Rückenelektrode aufweist. Die Frontelektrode und die Rückenelektrode sind jeweils mit einem Anschluss verbunden. An die Anschlussleiter wird eine Spannung angelegt. Dadurch sendet der eingebettete Leuchtstoff Licht aus, das durch die transparente Frontelektrode austritt.

Die elektrische Energie wird direkt in Licht umgewandelt, ohne dass störende Wärme entsteht, die das Meßergebnis verfälschen kann. Dadurch werden reproduzierbare Messergebnisse auch im Dauerbetrieb des Prüfgeräts erzeugt.

Darüber hinaus besteht der Vorteil, dass der in Folienform ausgebildete Elektrolumineszenz - Leuchtkörper im Querschnitt gesehen vorzugsweise parabelförmig oder ellipsenförmig gebogen werden kann, so dass auf der Probe ein völlig diffuses homogenes Messlicht erzeugt werden kann. Die Auswertung der Messergebnisse kann mit einem oberhalb der Probe im Scheitelpunkt des Elektrolumineszenz - Leuchtkörpers angeordneten Messwertaufnehmer vorgenommen werden, der einen fotoelektrischen Sensor und / oder eine optoelektronische Erfassungseinrichtung, gegebenenfalls mit einem optischen System, wie CCD - Videokamera, umfasst.

Die Erfindung soll nachstehend anhand eines Ausführungsbeispiels unter Bezugnahme auf die Zeichnungen näher erläutert werden. Vorteilhafte Ausgestaltungen der Erfindung ergeben sich aus den Unteransprüchen. Im einzelnen zeigt
- Figur 1: den Aufbau eines Prüfgerätes und
- Figur 2: die Darstellung des Meniskuszylinders und die Anordnung der Probe mit der elektrischen Lichtquelle in schematischer Darstellung

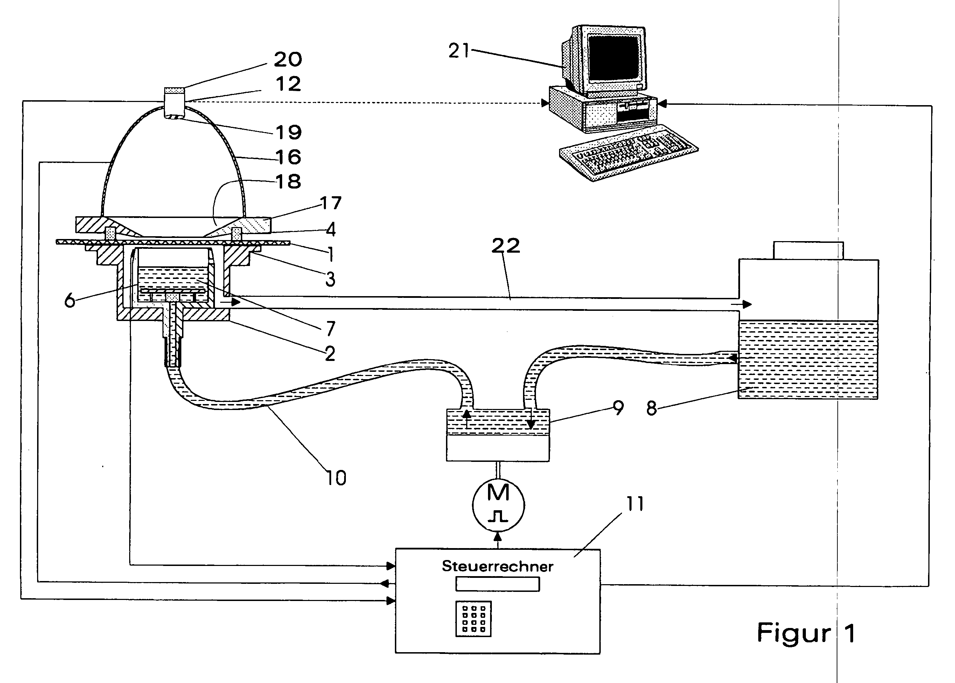

In Figur 1 ist eine zu prüfenden Probe 1 aus Papier, Pappe, Filtermaterial oder aus einem anderen saugfähigen Material dargestellt.

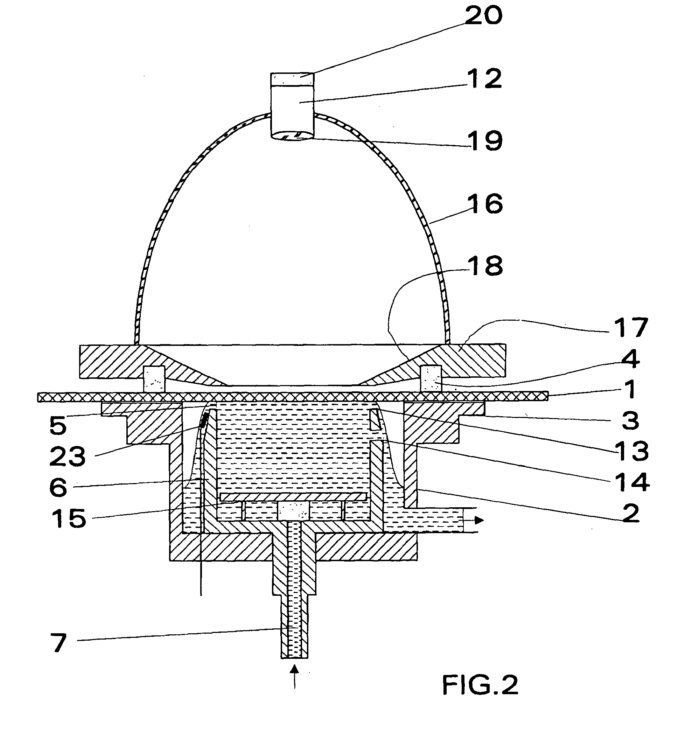

Die Probe 1 wird auf einen Überlaufzylinder 2 mit Probenauflage 3 aufgelegt und durch einen beispielsweise aus Moosgummi bestehenden Probenhaltering 4 gehalten. Während der Messung wird die, durch die ständige Erneuerung des in Figur 2 dargestellten Meniskus 5 im Meniskuszylinder 6 abfließende Prüfflüssigkeit 7 im Überlaufzylinder 2 aufgefangen und in einen Vorratsbehälter 8 zurückgeführt. Mit der steuerbaren Mikrodosierpumpe 9 wird das Prüfflüssigkeit 7 dem Vorratsbehälter 8 entnommen und über eine Zulaufleitung 10 dem Meniskuszylinder 6 zugeführt. Der Überlaufzylinder 2 ist mittels Rücklaufleitung 22 mit der Mikrodosierpumpe 9 verbunden ist. Die Steuerung erfolgt über den Steuerrechner 11.

Der Vorratsbehälter 8 dient der Aufnahme der Prüfflüssigkeit 7. Als Prüfflüssigkeit 7 sind Prüftinte nach DIN 53126, gefärbte Prüföle, aber auch stark saure oder alkalische Prüfmedien zugelassen. Das gesamte mit den Prüfmedien in Berührung kommende System ist entsprechend dimensioniert.

Der aus Moosgummi bestehende Probenhaltering 4 hält die während der Messung die auf dem Meniskus 5 schwimmende Probe 1 fest und dichtet den Messwertaufnehmer 12 gegen das Umgebungslicht ab.

Im Meniskuszylinder 6 wird nach Figur 2 während der Messung der Probe 1 ein Meniskus 5 erzeugt. Der Überlaufzylinder 2 ist mit der in der Prüfebene angeordnete Probenauflage 3 für die Probe 1 derart angeordnet, dass die Probenauflage 3 definiert gegenüber dem Überlaufrand 13 des Meniskuszylinders 6 annähernd um die Höhe des Meniskus 5 vertikal mit einem Versatz beabstandet ist. Die Höhe des Meniskus 5 reicht bei nicht aufgelegter Probe 1 über den Überlaufzylinder 2 mit Probenauflage 3 und wird mit der Ausgleichsdüse 14 und der steuerbaren Mikrodosierpumpe 9 eingestellt. Der Strömungsverteiler 15 sorgt für eine starke Beruhigung der einströmenden Prüfflüssigkeit 7, so dass sich der Meniskus 5 mit einer fast ebenen Oberfläche ausbildet.

Bei aufgelegter Probe 1 und ausgelöster Messung benetzt der Meniskus 5 die Probe 1 und die Prüfflüssigkeit 7 läuft über den Überlaufrand 13 des Meniskuszylinders 6 und erreicht einen am Überlaufrand 7 angeordneten Temperatursensor 23, der die Temperatur der Prüfflüssigkeit 7 misst und den Beginn der Messzeit zur Leimungsgradbestimmung signalisiert.

Wenn der Meniskus 5 der Prüfflüssigkeit 7 die Probe 1 benetzt hat, bildet sich an der Unterseite der Probe 1 eine laminare Radialströmung aus, die bedingt durch die parabolische Form an der Probenunterseite die Geschwindigkeit Null hat, d.h. auf die Probe 1 wird keine Kraft durch die Prüfflüssigkeit 7 ausgeübt, die das Messergebnis verfälscht. Durch die sich einstellende laminare Radialströmung können sich im Bereich des Meniskus 5 unter der Probenoberfläche auch keine das Messergebnis verfälschenden Luftbläschen einstellen. Durch die Saugwirkung der Probe 1 wird das parabolische Strömungsprofil infinitesimal gestört, so dass kleine Saugprofile in die Strömungsparabel geringfügig hineinreichen und zur Erneuerung der Prüfflüssigkeit 7 an der Unterseite der Probe 1 führen. Damit wird erreicht, dass die während der Messung Probe 1 wirkende Prüfflüssigkeit 7 immer die gleichen physikalischen und chemischen Eigenschaften aufweist.

Mit der vom Temperatursensor 23 gemessenen Temperatur wird z.B. die temperaturabhängige Oberflächenspannung der Prüfflüssigkeit 7 korrigiert.

Der Messwertaufnehmer 12 besteht aus einem Tubus, in dem ein fotoelektrischer Sensor oder ein Halbleiterstrahlungsempfänger mit einer Photodiode und / oder eine optoelektronische Erfassungseinrichtung mit einer CCD - Videokamera 20 und ein optisches System 19 befestigt sind. Das optische System 19 bildet die durch die Messblende 18 vorgegebene Messfläche auf den Halbleiterstrahlungsempfänger ab. Im Fall der Verwendung einer Videokamera 20 wird die Messblende 18 als Maßstab zur Berechnung der Größe unterschiedlich eingefärbter Partikel, hervorgerufen durch die Stoffformation bei Papieren, verwendet.

Die elektrische Lichtquelle wird durch einen in Folienform ausgebildeten Elektrolumineszenz - Leuchtkörper 16 gebildet, der im Querschnitt gesehen vorzugsweise parabelförmig oder ellipsenähnlich gebogen ist. Der Elektrolumineszenz - Leuchtkörper 16 ist in einer Halterung 17 der Probenauflage 3 gegenüberliegend befestigt, wobei die Halterung 17 schwenkbar gelagert oder abnehmbar angeordnet und mit der mit Messlicht beaufschlagten Messblende 18 versehen ist. Im Scheitelpunkt oberhalb der Probe 1 ist der Elektrolumineszenz - Leuchtkörper 16 mit dem Messwertaufnehmer 12 angeordnet.

Elektrolumineszenz - Leuchtkörper 16 sind Lambertstrahler, so dass die durch die Messblende 18 vorgegebene Messfläche völlig diffus beleuchtet wird. Mit der Form der Lichtquelle und der Anordnung des Halbleiterstrahlungsempfängers unter 0 ° wird die Messgeometrie d /0° nach DIN 5033 realisiert.

Abhängig von der Färbung der Probe 1 kann der Strahlungsbereich der Elektrolumineszenz - Leuchtkörper 16 gewählt werden, dabei kommen auch monochromatisch strahlende Elektrolumineszenz - Leuchtkörper 16 zum Einsatz. Elektrolumineszenz - Leuchtkörper 16 geben nur eine extrem geringe Wärme ab, so dass die Probe 1 während der Messung nicht erwärmt wird.

Die Messblende 18 begrenzt die Messfläche auf der Probe 1 auf ein vorgegebenes Maß. Sie ist aber auch Maßstab für verschiedene Berechnungen bei der Bildverarbeitung wie z. B. die Größe der unterschiedlich eingefärbten Partikel durch die Stoffformation bei Papieren und des Färbungsprofils der Stoffformation.

Der in Figur 1 dargestellte Steuerrechner 11 übernimmt die Bedienerführung über Display und Tastatur, die Steuerung des gesamten Messprozesses, die Messwertverarbeitung, die Berechnung des Leimungsgrades und die Anzeige der Messzeit und des Leimungsgrades auf dem Display.

Im Fall der Verwendung einer CCD - Videokamera 20 als Messwertaufnehmer 12 oder der Verarbeitung der Messwerte in einem Expertensystem, ist ein Personalcomputer 21 notwendig. In diesen Fällen wird die Standardschnittstelle des Steuerrechners 11 mit der Standardschnittstelle des Personalcomputers 21 verbunden.

An Hand der Messung des Leimungsgrades eines 120 Gramm Papiers mit Prüftinte nach DIN 53126 wird der Messvorgang erläutert.

Der Bediener hat die Probe 1 nach DIN 53102 vorbereitet und auf den Überlaufzylinder 2 mit Probenauflage 3 entsprechend Figur 1 gelegt. Das Prüfgerät ist eingeschaltet und der Bediener erhält über das Display des Steuerrechners 11 die Aufforderung das Flächengewicht des zu messenden Papiers in g/m² einzugeben.

Mit der Tastatur des Steuerrechners 11 wird durch den Bediener beispielsweise 120 eingegeben. Mit Betätigung der Taste ENTER wird die Eingabe des Flächengewichts abgeschlossen. Danach wird der Bediener aufgefordert, den Sollremissionsgrad Beta (Lambda) in Prozent einzugeben. Hierbei sind 50 % voreingestellt. Andere Werte zwischen 20 % und 80 % sind sinnvoll und möglich. Ist dies erfolgt, wird der Bediener aufgefordert die Messung auszulösen, wobei mit Betätigung der Taste ENTER die Messung beginnt. Der Steuerrechner 11 schaltet den Elektrolumineszenz - Leuchtkörper 16 ein und vom Messwertaufnehmer 12 wird der Remissionsgrad Beta (Lambda) der Probe 1 gemessen und gleich 100 % gesetzt und daraus der Sollremissionsgrad Beta (Lambda) zu 50 % berechnet. Bis zu diesem Zeitpunkt entspricht der Füllstand des Meniskuszylinders 6 dem in Figur 1 gezeichnetem Füllstand, der durch die Ausgleichsdüse 14 vorgegeben ist.

Nun wird die Mikrodosierpumpe 9 eingeschaltet und im Meniskuszylinder 6 wird entsprechend Figur 2 ein Meniskus 5 erzeugt, der die Probeunterseite der Probe 1 benetzt. Mit der Benetzung bildet sich ein laminares parabolisches Strömungsprofil mit radialer Strömungsrichtung aus, das sofort den Temperatursensor 23 erreicht, der die eigentliche Messung auslöst, die mit der Temperaturmessung beginnt.

In schneller Folge misst nun der Messwertaufnehmer 12 den Remissionsgrad Beta (Lambda) der Probe 1 und vergleicht ihn mit dem berechneten Sollremissionsgrad Beta 50 % (Lambda). Die Prüftinte durchdringt nun auf Grund der wirkenden Kapillarkräfte die Probe 1 und verfärbt diese, so dass sich der gemessene Remissionsgrad Beta (Lambda) verringert, bis er den Sollremissionsgrad Beta 50 % (Lambda) erreicht. Zu diesem Zeitpunkt bricht der Steuerrechner 11 die Messung ab, schaltet die Mikrodosierpumpe 9 und den Elektrolumineszenz - Leuchtkörper 16 aus und berechnet die Messzeit t 50 %, korrigiert diese bei Abweichung von der Normtemperatur, falls notwendig, und berechnet den Leimungsgrad z.B. nach einem Algorithmus des Instituts für grafische Technik Leipzig. Die Werte der Messzeit t 50 % und des Leimungsgrades bzw. der Leimungszahl werden auf dem Display des Steuerrechners 11 angezeigt. Die Messwerte können aber auch über Standardschnittstellen dem Personalcomputer 21 übergeben werden, der sie dann in einem Expertensystem verarbeitet und speichert.

Mit dem Abschalten der Mikrodosierpumpe 9 fließt die Prüfflüssigkeit 7 durch die Ausgleichsdüse 14 in den Überlaufzylinder 2 mit Probenauflage 3, der Füllstand im Meniskuszylinder 6 erniedrigt sich und eine neue Messung kann ausgelöst werden.

Wird anstelle einer Photodiode eine CCD - Videokamera 20 verwendet, wird diese mit der Bildverarbeitungskarte des Personalcomputers 21 und der Personalcomputer 21 über die Standardschnittstellen mit dem Steuerrechner 11 verbunden. Die Messung wird vom Personalcomputer 21 über den Steuerrechner 11 ausgelöst und erfolgt prinzipiell in gleicher Weise wie beschrieben.

Anstatt des Remissionsgrades Beta (Lambda) wird das Luminanzsignal Ly der CCD - Videokamera 20 für die Messung benutzt und so werden Bilder in schneller Folge aufgenommen und hinsichtlich des Luminanzsignals Ly mit dem zu 50 % bewerteten Luminanzsignals Ly des Bildes der unbenetzten Probe 1 ( Sollwert) verglichen. Mit Erreichen des Sollwertes speichert der Personalcomputer 21 das letzte Bild und bricht über den Steuerrechner 11 die Messung ab, berechnet die Messzeit t 50 % und Leimungsgrad bzw. Leimungszahl.

Zusätzlich werden mit Bildverarbeitungsprogrammen das Durchtränkungsprofil der Messfläche, das Rückschlüsse auf die Stoffformation zulässt, die statistische Verteilung der Luminanz über der Messfläche und die statistische Verteilung der unterschiedlich eingefärbten Partikel, hervorgerufen durch die Stoffformation in Papieren, über der Messfläche ermittelt und dargestellt.

## Patentansprüche

1. Vorrichtung zur fotoelektrischen Prüfung des Leimungsgrades von Papier und der Saugfähigkeit von Proben aus Papier, Pappe, Filtermaterial oder einem anderen saugfähigen Material mit Hilfe einer Prüfflüssigkeit sowie unter Verwendung einer horizontal in einer Prüfebene angeordneten und mit einem elastischen Druckring gehaltenen Probe, einer elektrischen Lichtquelle, eines Lichtempfänger, weiterhin umfassend ein ortsfestes, über eine Zulaufleitung (10) mit einer Mikrodosierpumpe (9) verbundenes zylinderförmiges Gefäß (6) für die Prüfflüssigkeit (7), wobei bei Inbetriebnahme der Mikrodosierpumpe (9) das zylinderförmige Gefäß (6) mit einem aus überlaufender Prüfflüssigkeit (7) bestehenden Meniskus (5) versehen und die Probe (1) auf dem Meniskus (5) schwimmend angeordnet ist, **gekennzeichnet durch** einen in Folienform ausgebildeten Elektrolumineszenz-Leuchtkörper (16).

2. Vorrichtung nach Anspruch 1, **dadurch gekennzeichnet, dass** das zylinderförmige Gefäß (6) in einem Überlaufzylinder (2) für Prüfflüssigkeit (7) angeordnet ist, wobei der Überlaufzylinder (2) mittels Rücklaufleitung (22) mit der Mikrodosierpumpe (9) verbunden ist.

3. Vorrichtung nach Anspruch 2, **dadurch gekennzeichnet, dass** in der Rücklaufleitung ein Vorratsbehälter (8) angeordnet ist.

4. Vorrichtung nach einem der Ansprüche 1 bis 3, **dadurch gekennzeichnet, dass** eine Ausgleichsdüse (14) in dem zylinderförmigen Gefäß (6) angeordnet ist.

5. Vorrichtung nach einem der Ansprüche 1 bis 4, **dadurch gekennzeichnet, dass** der Überlaufzylinder (2) eine in der Prüfebene angeordnete Probenauflage (3) für die Probe (1) aufweist, die definiert gegenüber dem Überlaufrand (13) des zylinderförmigen Gefäßes (6) annähernd um die Höhe des Meniskus (5) vertikal mit einem Versatz beabstandet ist.

6. Vorrichtung nach einem der Ansprüche 1 bis 5, **dadurch gekennzeichnet, dass** der Probenauflage (3) gegenüberliegend der auf der Probe (1) aufsitzende elastische Probenhaltering (4) mit einer Halterung (17) für **den Elektrolumineszenz-**Leuchtkörper (16) angeordnet ist, wobei die Halterung (17) schwenkbar gelagert oder abnehmbar angeordnet und mit einer mit Messlicht beaufschlagten Messblende (18) versehen ist.

7. Vorrichtung nach einem der Ansprüche 1 bis 6, **dadurch gekennzeichnet, dass** der in Folienform ausgebildete Elektrolumineszenz - Leuchtkörper (16) im Querschnitt gesehen parabelförmig oder ellipsenähnlich gebogen ist.

8. Vorrichtung nach einem der Ansprüche 1 bis 7, **dadurch gekennzeichnet, dass** der Elektrolumineszenz - Leuchtkörper (16) im Scheitelpunkt oberhalb der Probe (1) mit einem mit einem Steuerrechner (11) verbundenen Lichtempfänger (12) versehen ist.

9. Vorrichtung nach Anspruch 8, **dadurch gekennzeichnet, dass** der Lichtempfänger (12) einen fotoelektrischen Sensor oder einen Halbleiterstrahlungsempfänger mit einer Photodiode oder eine optoelektronische Erfassungseinrichtung mit einer CCD - Videokamera (20) enthält.

10. Vorrichtung nach Anspruch 9, **dadurch gekennzeichnet, dass** die CCD - Videokamera (20) mit der Bildverarbeitungskarte des Personalcomputers (21) und der Personalcomputer (21) über die Standardschnittstellen mit dem Steuerrechner (11) verbunden ist.

11. Vorrichtung nach einem der Ansprüche 1 bis 10, **dadurch gekennzeichnet, dass** der Elektrolumineszenz - Leuchtkörper (16), der Lichtempfänger (12) und die Mikrodosierpumpe (9) mit dem Steuerrechner (11) zur Steuerung einer automatischen fotoelektrischen Ausmessung verbunden sind.

12. Vorrichtung nach einem der Ansprüche 1 bis 11, **dadurch gekennzeichnet, dass** in dem Überlaufzylinder (2) ein die Temperatur der Prüfflüssigkeit (7) messender Temperatursensor (23) angeordnet ist.

13. Vorrichtung nach Anspruch 12, **dadurch gekennzeichnet, dass** der Temperatursensor (23) schaltungstechnisch mit dem Steuerrechner (11) verbunden ist, wobei ein Steuersignal für den Beginn der Messzeit zur Leimungsgradbestimmung abgegeben wird.

## Claims

1. Device for photoelectrically testing the degree of gluing of paper and the absorbency of samples consisting of paper, cardboard, filter material or any other absorbent material with the aid of a testing fluid (7) and using a sample (1) arranged horizontally on one sample level and held with an elastic sample mount (4), an electrical source of light (16), a light receiver (12), continue comprehensively an stationary cylinder-shaped vessel (6) connected via feed line (10) with a microfeeding pump (9) for the testing fluid (7) where the cylinder-shaped vessel (6) has a meniscus lens (5) consisting of overflowing testing fluid (7) when starting up the microfeeding pump (9) and the sample (1) is arranged to be floating on the meniscus lens (5), **characterised by** an electroluminescence illuminant (16) formed in foil shape.

2. Device in accordance with claim 1 **characterised by** the fact that the cylinder-shaped vessel (6) is arranged in an overflow cylinder (2) for the testing fluid (7) where the overflow cylinder (2) is connected with the microfeeding pump (9) with a return tube (22).

3. Device in accordance with claim 2 **characterised by** the fact that a reservoir (8) is arranged in the return tube.

4. Device in accordance with one of the claims 1 through 3 **characterised by** the fact that an equalising jet (14) is arranged in the cylinder-shaped vessel (6).

5. Device in accordance with one of the claims 1 through 4 **characterised by** the fact that the overflow cylinder (2) has a sample support (3) for the sample (1) arranged on the testing level that has a vertical distance defined to the overflow edge (13) of the cylinder-shaped vessel (6) with an offset about the approximate height of the meniscus lens (5).

6. Device in accordance with one of the claims 1 through 5 **characterised by** the fact that the sample support (3) is arranged opposite the elastic sample mount (4) attached to the sample (1) with a mount (17) for the electroluminescence illuminant (16) where the mount (17) is lodged to be pivoting or arranged to be removable and has a measuring diaphragm (18) impinged by measuring light.

7. Device in accordance with one of the claims 1 through 6 **characterised by** the fact that the electroluminescence illuminant (16) formed in foil shape is bent in a parabolic shape or similar to an ellipse seen in the cross-section.

8. Device in accordance with one of the claims 1 through 7 **characterised by** the fact that the electroluminescence illuminant (16) has a light receiver (12) connected to a control computer (11) at the zenith above the sample (1).

9. Device in accordance with claim 8 **characterised by** the fact that the light receiver (12) contains a photoelectric sensor or a semiconductor radiation receiver with a photodiode or optoelectronic recording equipment with a CCD video camera (20).

10. Device in accordance with claim 9 **characterised by** the fact that the CCD video camera (20) is connected with the image processing card of a personal computer (21) and the personal computer (21) is connected with the control computer (11) via standard interfaces.

11. Device in accordance with one of the claims 1 through 10 **characterised by** the fact that the electroluminescence illuminant (16), the light receiver (12) and the microfeeding pump (9) are connected with control computer (11) for controlling automatic photoelectric measurement.

12. Device in accordance with one of the claims 1 through 11 **characterised by** the fact that a temperature sensor (23) measuring the temperature of the testing fluid (7) is arranged in the overflow cylinder (2).

13. Device in accordance with claim 12 **characterised by** the fact that the temperature sensor (23) is connected via switches with the control computer (11) where a control signal is emitted for the beginning of the measuring period for determining the degree of gluing.

## Revendications

1. Dispositif de contrôle photoélectrique du degré de collage de papier et de la capillarité d'échantillons en papier, en carton, en matériau filtrant ou d'un autre matériau absorbant à l'aide d'un liquide d'essai (7) ainsi qu'en utilisant un échantillon (1) disposé à l'horizontal, se présentant dans un plan d'essai et avec un anneau de blocage d'essai élastique (4), une source lumineuse électrique (16) et un récepteur de lumière (12), comportant en outre un réservoir stationnaire cylindrique (6) pour le liquide d'essai (7) relié via une conduite d'arrivée (10) avec une pompe à microdosage (9). Ledit réservoir cylindrique (6), lorsque la pompe à microdosage (9) est mise en marche, est pourvu d'un ménisque (5) se composant d'un liquide d'essai débordant (7) et l'échantillon (1) est disposé flottant sur le ménisque (5), **caractérisé par** un élément lumineux électroluminescent (16) en forme de films.

2. Dispositif selon la revendication 1, **caractérisé en ce que** le réservoir (6) cylindrique est disposé dans une vanne de trop-plein (2) pour le liquide d'essai (7). Ladite vanne de trop-plein (2) est raccordée à la pompe de microdosage (9) à l'aide de la conduite de retour (22).

3. Dispositif selon la revendication 2, **caractérisé en ce qu'**un réservoir de stockage (8) est disposé dans la conduite de retour.

4. Dispositif selon une des revendications 1 à 3, **caractérisé en ce qu'**un gicleur d'équilibre (14) est disposé dans le récipient cylindrique (6).

5. Dispositif selon une des revendications 1 à 4, **caractérisé en ce que** la vanne de trop-plein (2) présente un logement à échantillon (3) disposé dans le plan d'essai pour l'échantillon (1) qui, situé de manière définie par rapport au bord de trop-plein (13) du réservoir (6) cylindrique de trop-plein, est espacé avec un décalage à la verticale presqu'à la hauteur du menisque (5).

6. Dispositif selon une des revendications 1 à 5, **caractérisé en ce que** le logement à échantillon (3) est disposé à l'opposé de l'anneau de blocage d'essai élastique (4) reposant sur l'échantillon (1) avec une fixation (17) pour la élément lumineux électroluminescent (16), laquelle fixation (17) est montée de manière à pouvoir pivoter ou placée de façon amovible et pourvue d'un diaphragme de mesure (18) alimenté avec une lumière de mesure.

7. Dispositif selon une des revendications 1 à 6, **caractérisé en ce que** le élément lumineux électroluminescent (16) conçu en forme de films est courbé, vue en section transversale, en forme de parabole ou elliposaïdale.

8. Dispositif selon une des revendications 1 à 7, **caractérisé en ce que** le corps lumineux (16) électroluminescent est pourvu au sommet en amont de l'échantillon (1) d'un récepteur de lumière (12) relié à un ordinateur de commande (11).

9. Dispositif selon la revendication 8, **caractérisé en ce que** le récepteur de lumière (12) comprend un détecteur photoélectrique ou un détecteur de radiations semi-conducteur avec une photodiode ou un dispositif de détection optoélectronique avec une caméra CCD (20).

10. Dispositif selon la revendication 9, **caractérisé en ce que** la caméra CCD (20) est connectée à une carte de traitement d'images d'un ordinateur personnel (21), lequel ordinateur personnel (21) est relié à l'ordinateur de commande (11) via des interfaces standards.

11. Dispositif selon une des revendications 1 à 10, **caractérisé en ce que** le élément lumineux électroluminescent (16), le récepteur de lumière (12) et la pompe à microdosage (12) sont connectés à l'ordinateur de commande (11) pour commander une mesure photoélectrique automatique.

12. Dispositif selon une des revendications 1 à 11, **caractérisé en ce qu'**un capteur de températures (23), mesurant la température du liquide d'essai (7), est disposé dans la vanne de trop-plein (2).

13. Dispositif selon la revendication 12, **caractérisé en ce que** le capteur de températures (23) est relié à l'ordinateur de commande (11) au niveau de la technique de circuit, comprenant un signal de commande délivré pour le début du temps de comptage de la détermination du degré de collage.
